# EUROPEAN PATENT SPECIFICATION

(11) **EP 3 950 942 B1**
(45) Date of publication and mention of the grant of the patent: **29.04.2026**
(21) Application number: 20783536.4
(22) Date of filing: 03.04.2020
(51) Int. Cl.: C12N 15/09, C12N 15/90, C12N 15/10, C12N 15/113

(54) **METHOD FOR PRODUCING KNOCK-IN CELL**
VERFAHREN ZUR HERSTELLUNG EINER KNOCK-IN ZELLE
PROCÉDÉ DE PRODUCTION DE CELLULES KNOCK-IN

(30) Priority: 05.04.2019 JP 2019072782
(43) Date of publication of application: 09.02.2022
(73) Proprietor: Osaka University, Suita-shi, Osaka 565-0871 (JP)
(72) Inventor: MASHIMO, Tomoji, Suita-shi, Osaka 565-0871 (JP); YOSHIMI, Kazuto, Suita-shi, Osaka 565-0871 (JP); UNO, Yoshihiro, Suita-shi, Osaka 565-0871 (JP); KOTANI, Yuko, Suita-shi, Osaka 565-0871 (JP); MIYASAKA, Yoshiki, Suita-shi, Osaka 565-0871 (JP); OKA, Yuichiro, Suita-shi, Osaka 565-0871 (JP); SATO, Makoto, Suita-shi, Osaka 565-0871 (JP)
(74) Representative: J A Kemp LLP
(86) International application number: PCT/JP2020/015291
(87) International publication number: WO 2020/204159

(56) References cited:
- WO-A1-2016/080399
- WO-A1-2018/097257
- JP-A- 2016 537 982
- US-A1- 2017 198 302
- XIANGJUN HE ET AL: "Knock-in of large reporter genes in human cells via CRISPR/Cas9-induced homology-dependent and independent DNA repair", NUCLEIC ACIDS RESEARCH, vol. 44, no. 9, 4 February 2016 (2016-02-04), GB, pages e85 - e85, XP055415869, ISSN: 0305-1048, DOI: 10.1093/nar/gkw064
- HE XIANGJUN ET AL: "New Turns for High Efficiency Knock-In of Large DNA in Human Pluripotent Stem Cells", vol. 2018, 3 July 2018 (2018-07-03), US, pages 1 - 7, XP093006320, ISSN: 1687-966X, Retrieved from the Internet <URL:http://downloads.hindawi.com/journals/sci/2018/9465028.xml> DOI: 10.1155/2018/9465028
- GRATZ, SCOTT J. ET AL.: "Genome Engineering of Drosophila with the CRISPR RNA-guided Cas9 Nuclease", GENETICS, vol. 194, 2013, pages 1029 - 1035, XP055102690, DOI: 10.1534/genetics.113.152710
- YAO, XUAN ET AL.: "Homology-mediated end joining- based targeted integration using CRISPR/Cas9", CELL RES., vol. 27, 2017, pages 801 - 814, XP055516046, DOI: 10.1038/cr.2017.76
- YAO, XUAN ET AL.: "Tild-CRISPR Allows for Efficient and Precise Gene Knockin in Mouse and Human Cells", DEV. CELL, vol. 45, 2018, pages 526 - 536, XP055648098
- ZHANG, JIAN-PING ET AL.: "Efficient precise knockin with a double cut HDR donor after CRISPR/Cas9-mediated double-stranded DNA cleavage", GENOME BIOL., vol. 18, no. 35, 2017, pages 1 - 18, XP055399694
- AIDA, TOMOMI ET AL.: "Cloning-free CRISPR/Cas system facilitates functional cassette knockin in mice", GENOME BIOL., vol. 16, no. 87, 2015, pages 1 - 11, XP021215503
- MIYASAKA, YOSHIKI ET AL.: "CLIGK: one-step generation of conditional knockout mice", BMG GENOMICS, vol. 19, no. 318, 2018, pages 1 - 8, XP021256013, DOI: 10.1186/s12864-018-4713-y
- KAZUTO YOSHIMI, YOSHIKI MIYASAKA, YUKO KOTANI, KOSUKE HATTORI, ARISA TANIGAWA, YUKO YAMAUCHI, YOSHIHIRO UNO; TOMOJI MASHIMO: "S1-3(P-35) Combi-CRISPR: A novel strategy for efficient gene knock-in rodents", ABSTRACTS OF 4TH ANNUAL MEETING OF THE JAPANESE SOCIETY FOR GENOME EDITING; JUNE 3-5, 2019, 3 June 2019 (2019-06-03), pages S1-3(P-35), XP009531707
- UNO, YOSHIHIRO: "4P-0671 Generation of long-chain DNA knock-in mouse using CRISPR-Gas9 system", 42ND ANNUAL ACADEMIC CONFERENCE OF THE MOLECULAR BIOLOGY SOCIETY OF JAPAN; DECEMBER 3-6, 2019, vol. 42, 7 November 2019 (2019-11-07), pages 4P-0671, XP009531706

## Description

### [Technical Field]

The present invention relates to an *in vitro* method for producing a cell in which a donor sequence is inserted into a genome-editing target region on a genomic DNA, and use of a kit or composition in the method.

### [Background Art]

Genome editing technologies, such as zinc finger nucleases (ZFN), TAL effector nucleases (transcription activator-like effector nuclease; TALEN), and CRISPR-Cas9, are technologies that specifically cleave genomic DNA sequences in animal and plant cells and use intrinsic repair mechanisms to freely rewrite them into certain sequences. The use thereof is rapidly expanding in not only bioscience researches but also selective breeding of agricultural crops and livestock animals, regenerative medicine, and genome-editing therapy.

When CRISPR/Cas9 (NPL 1) is introduced into a fertilized mouse egg, a guide RNA binds to the target DNA sequence, and Cas9, which forms a complex with the guide RNA, causes a double-strand break in the target DNA. A DNA repair mechanism called non-homologous end joining (NHEJ) is then activated at the break site. By introducing genetic a mutation into the genomic DNA of the fertilized mouse egg through this DNA repair mechanism, a knock-out mouse can be produced. On the other hand, if a donor DNA with a sequence homologous to the genomic DNA is introduced into a fertilized mouse egg together with CRISPR/Cas9, a knock-in mouse with recombinant genes can be produced through homologous recombination (HR).

In 2013, knock-out and knock-in mice produced using CRISPR/Cas9 were reported (NPLs 2 and 3). Currently, gene knock-out can be performed very efficiently, but knock-in is still less efficient in cells and fertilized eggs.

Recently, various methods other than knock-in by homologous recombination using conventional double-stranded DNA donor vectors have been developed. For example, it has been reported that single-stranded oligo donor DNA (ssODN), together with CRISPR/Cas9, can be injected into cells or fertilized eggs to efficiently knock-in a donor sequence of one to several tens of bases (NPLs 4 and 5). The present inventors have also developed the 2-Hit 2-Oligo (2H2OP) method, which can knock-in plasmids of several thousands of bases or BAC vectors of 200000 bases by cleaving the target DNA sequence and donor vector with Cas9 and joining the cleavage sites with two ssODNs (NPL 6). Furthermore, there have been reported the production of conditional mice and GFP reporter knock-in mice with the Easi-CRISPR method using long single-stranded donor DNA (NPL 7) and the CLICK method using a combination of long single-stranded donor DNA with electroporation method (NPL 8). In addition, reported knock-in methods using double-stranded donor DNA include the cloning-free method using Cas9 protein and bimolecular guide RNA (NPL 9), the PITCH method using the MMEJ repair mechanism by microhomology (NPL 10), the HITI method of non-homologous end joining-dependent knock-in (NPL 11), the HMEJ method of homology sequence-dependent knock-in (NPL 12), the Tild-CRISPR method (NPL 13), and the like.

Although these methods have been reported as being capable of efficiently knocking-in several thousands of bases of DNA in cells, they may be inefficient or incapable of accurate knock-in in animal fertilized eggs. This is because in mammalian cells and fertilized eggs, non-homologous end joining predominates in double-stranded DNA break repair, and repair by homologous recombination occurs only rarely.

### [Citation List]

### [Non Patent Literature]

[NPL 1] Jinek M, et al., Science. 337(6096): 816-21 (2012)
[NPL 2] Yang H, et al., Cell. 154(6): 1370-9 (2013)
[NPL 3] Wang H, et al., Cell. 153(4): 910-8 (2013)
[NPL 4] Sander JD, et al., Nat Biotechnol. 32(4): 347-55 (2014)
[NPL 5] Yoshimi K, et al., Nat Commun. 5: 4240 (2014)
[NPL 6] Yoshimi K, et al., Nat Commun. 7: 10431 (2016)
[NPL 7] Quadros RM, et al., Genome Biol. 18(1): 92 (2017)
[NPL 8] Miyasaka Y, et al., BMC Genomics. 19(1): 318 (2016)
[NPL 9] Aida T, et al., Genome Biol. 16: 87 (2015)
[NPL 10] Sakuma T, et al., Nat Protoc. 11(1): 118-33 (2016)
[NPL 11] Suzuki K, et al., Nature. 540(7631): 144-9 (2016)
[NPL 12] Yao X, et al., Cell Res. 27(6): 801-14 (2017)
[NPL 13] Yao X, et al., Dev Cell. 45(4): 526-36 (2018)
[NPL 14] He X, et al., Nucleic Acids Res. May 19;44(9):e85 (2016). NPL 14 describes knock-in of large reporter genes in human cells via CRISPR/Cas9-induced homology-dependent and independent DNA repair.
[NPL 15] He X, et al., Stem Cells Int. Jul 3;2018:9465028 (2018). NPL 15 describes advances in developing novel targeting strategies for high efficiency knock-in of large DNA in human ESC/iPSC.

### [Summary of Invention]

### [Technical Problem]

The present invention has been made in view of such circumstances, and an object thereof is to provide a genome editing method capable of inserting long strands of donor DNA into a genome with higher efficiency and accuracy than has been previously possible. A further object described herein is to provide use of a kit and composition in the genome editing method.

### [Solution to Problem]

The present inventors have made earnest studies to achieve the above objects, and have found as a result that the use of a site-specific nuclease system which includes a combination of a molecule that simultaneously targets and cleaves one homology arm sequence of the donor DNA and the genomic sequence corresponding to the homology arm sequence, and a molecule that targets and cleaves the genomic region in the vicinity of the cleavage site by that molecule causes repair between genomic DNA and donor DNA through both non-homologous end joining and homologous recombination, making it possible to produce cells and organisms with knocked-in long donor sequences with high efficiency and accuracy. Thus, the present invention has been completed.

More specifically, the present invention provides:
An *in vitro* method for producing a cell in which a donor sequence is inserted into a genome-editing target region on a genomic DNA, comprising the step of:
introducing a site-specific nuclease system and donor DNA into a cell, wherein
the donor DNA is a DNA containing a nucleotide sequence in which a 5'-side homology arm sequence, a donor sequence, and a 3'-side homology arm sequence are arranged in this order from a 5'-side,
the site-specific nuclease system is a system that targets and cleaves sequences (i) to (iii) of group A or group B below, and the sequences (i) and (ii) are targeted by the same molecules constituting the site-specific nuclease system, and the sequence (iii) is targeted by other molecules constituting the site-specific nuclease system:
   Group A:
      (i) the 5'-side homology arm sequence
      (ii) a sequence in the genome-editing target region corresponding to the sequence (i)
      (iii) a sequence in the genome-editing target region between a sequence corresponding to the 5'-side homology arm sequence and a sequence corresponding to the 3'-side homology arm sequence; and
   Group B
      (i) the 3'-side homology arm sequence
      (ii) a sequence in the genome-editing target region corresponding to the sequence (i)
      (iii) a sequence in the genome-editing target region between a sequence corresponding to the 5'-side homology arm sequence and a sequence corresponding to the 3'-side homology arm sequence,
wherein the site-specific nuclease system is:
   a CRISPR/Cas system containing a combination of a guide RNA that targets the sequences (i) and (ii) and a guide RNA that targets the sequence (iii), or
   an artificial nuclease system containing a combination of: a fusion protein that contains a DNA-binding domain that targets the sequences (i) and (ii) and a nuclease domain, and a fusion protein that contains a DNA-binding domain that targets the sequence (iii) and a nuclease domain,
   wherein the method integrates the donor nucleic acid by the combinational repair of non-homologous end joining of the cleaved sequences (i) and (ii) and homologous recombination of the non-cleaved homology arm with the genomic sequence, wherein the method is not a process for modifying the germline genetic identity of human beings.

The invention also provides use of a kit or composition in the method of the invention, wherein the kit comprises:
a site-specific nuclease system and donor DNA, wherein
the donor DNA is a DNA containing a nucleotide sequence in which a 5'-side homology arm sequence, a donor sequence, and a 3'-side homology arm sequence are arranged in this order from a 5'-side,
the site-specific nuclease system is a system that targets and cleaves sequences (i) to (iii) of group A or group B below, and the sequences (i) and (ii) are targeted by the same molecules constituting the site-specific nuclease system, and the sequence (iii) is targeted by other molecules constituting the site-specific nuclease system,
   Group A:
      (i) the 5'-side homology arm sequence
      (ii) a sequence in the genome-editing target region corresponding to the sequence (i)
      (iii) a sequence in the genome-editing target region between a sequence corresponding to the 5'-side homology arm sequence and a sequence corresponding to the 3'-side homology arm sequence
   Group B
      (i) the 3'-side homology arm sequence
      (ii) a sequence in the genome-editing target region corresponding to the sequence (i)
      (iii) a sequence in the genome-editing target region between a sequence corresponding to the 5'-side homology arm sequence and a sequence corresponding to the 3'-side homology arm sequence
wherein the site-specific nuclease system is:
   a CRISPR/Cas system containing a combination of a guide RNA that targets the sequences (i) and (ii) and a guide RNA that targets the sequence (iii), or
   an artificial nuclease system containing a combination of: a fusion protein that contains a DNA-binding domain that targets the sequences (i) and (ii) and a nuclease domain, and a fusion protein that contains a DNA-binding domain that targets the sequence (iii) and a nuclease domain.

The invention is defined in the appended claims. Any aspects, embodiments and examples of the present disclosure which do not fall within the scope of the appended claims do not form part of the invention and are merely provided for illustrative purposes.

### [Advantageous Effects of Invention]

In conventional methods as well, insertion of a donor sequence of one to several tens of bases in length into a genome is possible by using a single-stranded oligo donor DNA (ssODN), and insertion of a donor sequence of several tens to several hundreds of bases into a genome is possible by using a long single-stranded donor DNA. However, efficient insertion of a donor sequence of several hundreds to several tens of thousands of bases in length into the genome is difficult with these methods, and the efficiency and accuracy of knock-in decreases as the single-stranded donor DNA becomes longer. On the other hand, knock-in by homologous recombination using conventional double-stranded DNA donor vectors has high accuracy but low knock-in efficiency. The PITCH method, HITI method, and HMEJ method basically rely on non-homologous end joining, resulting in random integration, multicopy, and deletion mutations, which reduce accuracy.

The present invention has made it possible to insert a donor sequence into the genome with higher efficiency and accuracy than conventional methods, with the breakthrough idea of combining repair by non-homologous end joining, which is significantly efficient, and repair by homologous recombination, which is less efficient but more accurate.

Many of the donor sequences that are currently most in need of knock-in, such as site-specific recombination systems (Cre-loxP system, CreERT2-loxP system, FLP-FRT system) and various genes linked to promoters, are more than 2000 bases long, but the present invention can demonstrate high efficiency and accuracy even when the donor sequences are long.

### [Brief Description of Drawings]

[Fig. 1] Fig. 1 is a diagram showing the principle of the genome editing system described herein, which uses combinational repair of non-homologous end joining and homologous recombination.
[Fig. 2A] Fig. 2A is a diagram showing the production of Kcnab1-ERT2-iCreERT2 mice using the method described herein.
[Fig. 2B] Fig. 2B is a photograph showing the results of genotyping of the genome-editing target region in F1 mice obtained by crossing F0 mice produced by the method in Fig. 2A.
[Fig. 3A] Fig. 3A is a diagram showing the production of Ctgf-ERT2-iCreERT2 mice using the method described herein.
[Fig. 3B] Fig. 3B is a photograph showing the results of genotyping of the genome-editing target region in F1 mice obtained by crossing F0 mice produced by the method in Fig. 3A.
[Fig. 4A] Fig. 4A is a diagram showing the production of Tp53-CAG-CFP rats using the method described herein.
[Fig. 4B] Fig. 4B is a photograph showing the results of genotyping of the genome-editing target region in F0 rats produced by the method of Fig. 4A.
[Fig. 4C] Fig. 4C is a photograph showing the results of detecting the expression of fluorescent protein (CFP) in F0 rats produced by the method of Fig. 4A.

### [Detailed Description]

Described herein is a method for producing a cell in which donor DNA is inserted into a genome-editing target region on genomic DNA.

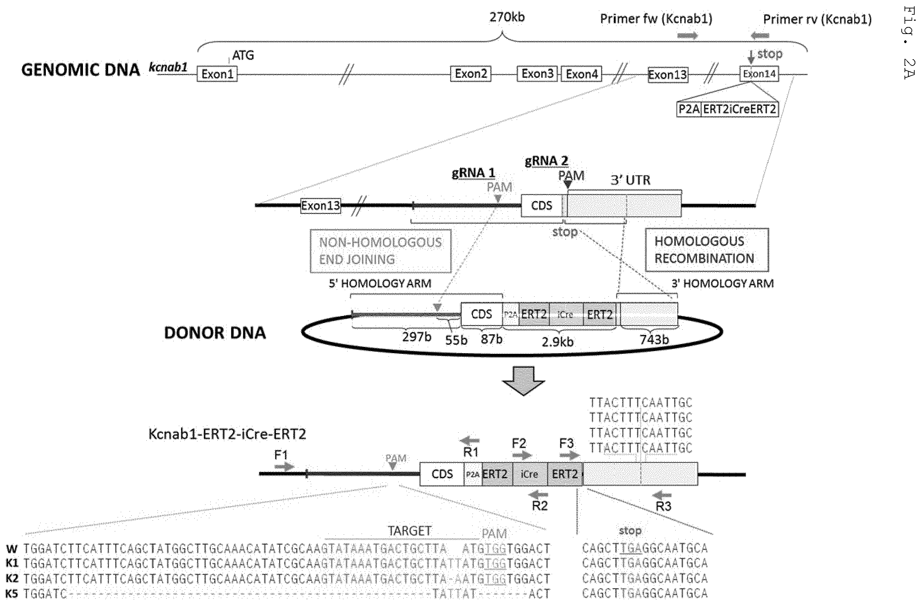

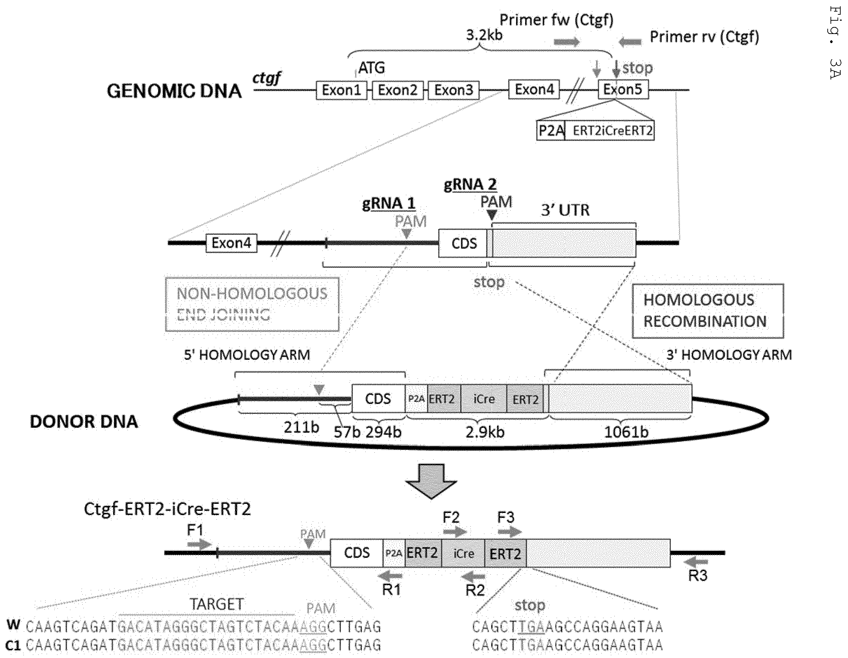

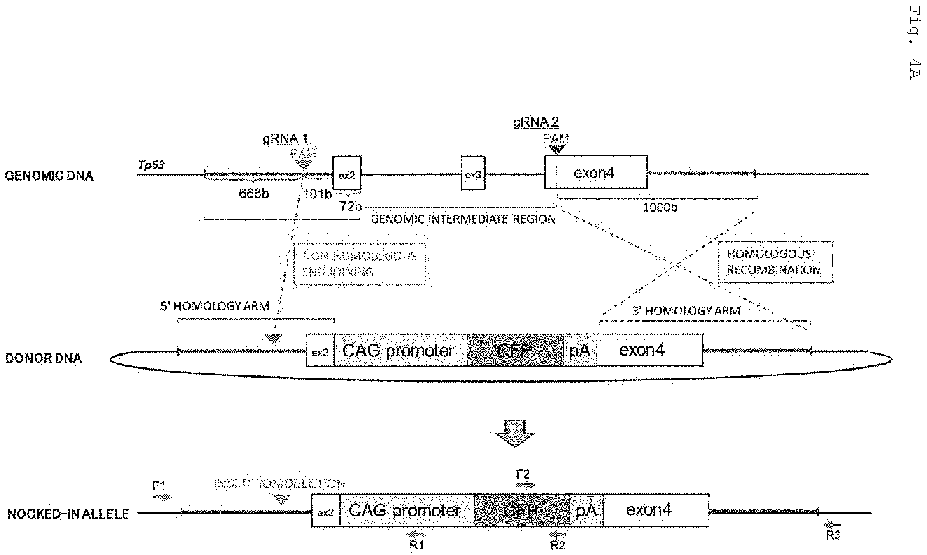

As used herein, "genome-editing target region" means the genomic region corresponding to the 5'-side homology arm of the donor DNA (hereinafter referred to as the "5'-side homology arm-corresponding genomic region"), the genomic region corresponding to the 3'-side homology arm of the donor DNA (hereinafter referred to as the "3'-side homology arm-corresponding genomic region"), and the intermediate region sandwiched between these genomic regions (hereinafter referred to as the "genomic intermediate region") (see, for example, Fig. 4A). When the 5'-side homology arm and 3'-side homology arm of the donor DNA are set for two consecutive regions on the genomic DNA, there is no genomic intermediate region (see, for example, Figs. 2A and 3A). There are no particular restrictions on the length of the genomic intermediate region, as long as genome editing by the method described herein is possible, but for example, it is 30000 bases long or less (for example, 10000 bases long or less, 5000 bases long or less, 3000 bases long or less, 1000 bases long or less, 500 bases long or less, 300 bases long or less, 100 bases long or less, 50 bases long or less, 30 bases long or less, 10 bases long or less, 5 bases long or less, 3 bases long or less). As the genome-editing target region, any region on the genomic DNA held by the cell to be genome-edited can be selected.

The method described herein involves the step of introducing a site-specific nuclease system and donor DNA into a cell.

There are no particular restrictions on the "site-specific nuclease system" used, as long as the system can specifically target and cleave any site on genomic DNA. The site-specific nuclease system can be a guide RNA-guided nuclease system or an artificial nuclease system. A guide RNA-guided nuclease system is a system containing a guide RNA that targets an arbitrary site on genomic DNA and a protein having nuclease activity, for example, a CRISPR/Cas system. An artificial nuclease system, on the other hand, is a system that utilizes a fusion protein that contains a DNA-binding domain and a nuclease domain, for example, the TALEN system, the ZFN system, or the PPR system.

Among the site-specific nuclease systems, a CRISPR/Cas system is preferable from the viewpoint that it uses nucleic acid (guide RNA) for the recognition of the target DNA sequence, thus allowing more freedom in selecting the target DNA sequence and simpler preparation thereof. CRISPR/Cas systems include, for example, the CRISPR/Cas9 system, the CRISPR/Cpf1 (Cas12a) system, and the CRISPR/Cas3 system.

A CRISPR/Cas system includes a Cas component and a guide RNA component. The Cas component can be, for example, in the form of a protein, in the form of mRNA encoding the protein, or in the form of a vector expressing the protein. In addition, the origin thereof is irrelevant. The guide RNA component can be, for example, in the form of RNA, or in the form of a vector expressing the RNA.

Cas components and guide RNA components can be produced by known technologies (such as PCR, restriction enzyme cleavage, DNA linkage technology, in vitro transcription and translation technology, and recombinant protein production technology) or can be purchased. The amino acid sequences of Cas components and the nucleotide sequences encoded by them can be obtained from the literature or published databases (such as GenBank; http://www.ncbi.nlm.nih.gov).

In the CRISPR/Cas9 system, the Cas9 protein forms a complex with a guide RNA composed of crRNA and tracrRNA, and the complex is targeted to the target DNA sequence to cleave the target DNA (for example, International Publication No. 2014/093712, International Publication No. 2013/176772, International Publication No. 2013/142578, and the like). The guide RNA can be in the form of two molecules composed of crRNA fragments and tracrRNA fragments, or in the form of a single molecule (sgRNA) in which crRNA and tracrRNA are combined via an intervening sequence. In the CRISPR/Cpf1 system, the Cpf1 protein forms a complex with crRNA, which is targeted to the target DNA sequence to cleave the target DNA (for example, International Publication No. 2016/205711 and the like). Also, in the CRISPR/Cas3 system, the Cas3 protein and Cascade protein form a complex with crRNA, which is targeted to the target DNA sequence to cleave the target DNA (for example, International Publication No. 2018/225858 and the like) .

Note that newly introduced CRISPR/Cas systems, such as the CRISPR/Cas12b system and the CRISPR/CasX (Cas12e) system, can be used as well (Strecker J, et al., Nature Communications 10: 212 (2019), Liu JJ, et al., Nature 566: 218-223 (2019)).

The sequence of the portion of the crRNA sequence that binds to the target DNA has, for example, 90% or more, preferably 95% or more, more preferably 98% or more, further preferably 99% or more, and particularly preferably 100% identity with the target DNA sequence. The sequence identity can be calculated using BLAST or the like (for example, default parameters).

For the targeting of a CRISPR/Cas system to the target DNA sequence, the Cas protein needs to recognize a PAM (proto-spacer adjacent motif) sequence adjacent to the target DNA sequence. The PAM sequence can vary depending on the type and origin of the Cas protein. For typical PAM sequences, for example, in the S. pyogenes-derived Cas9 protein (type II), it is "5'-NGG," in the S. solfataricus-derived Cas9 protein (type I-A1), it is "5'-CCN," in the S. solfataricus-derived Cas9 protein (type I-A2), it is "5'-TCN," in the H. walsbyl-derived Cas9 protein (type I-B), it is "5'-TTC," in the E.coli-derived Cas9 protein (type I-E), it is "5'-AWG," in the E.coli-derived Cas9 protein (type I-F), it is "5'-CC," in the P. aeruginosa-derived Cas9 protein (type I-F), it is "5'-CC," in the S. Thermophilus-derived Cas9 protein (type II-A), it is "5'-NNAGAA," in the S. agalactiae-derived Cas9 protein (type II-A), it is "5'-NGG," in the S. aureus-derived Cas9 protein, it is "5'-NGRRT" or "5'-NGRRN," in the N. meningitidis-derived Cas9 protein, it is "5'-NNNNGATT," and in the T. denticola-derived Cas9 protein, it is "5'-NAAAAC." In Cpf1, it is typically "5'-TTN" or "5'-TTTN." In the Cse1 (Cas8) protein (a type of Cascade protein that constitutes the CRISPR-Cas3 system of type I-E), it is typically "5'-AAG," "5'-TAG," "5'-AAC," "5'-GAG," "5'-AGG," and "5'-ATG," and in the Cas5d protein (a type of Cascade protein that constitutes the CRISPR-Cas3 system of type I-D), it is typically "5'-GTH" (H is A, C, or T) (International Publication No. 2018/225858, International Publication No. 2019/039417) .

Note that it is also possible to modify PAM recognition by modifying the Cas protein (for example, introducing mutations) (Benjamin, P. et al., Nature 523, 481-485 (2015), Hirano, S. et al., Molecular Cell 61, 886-894 (2016), International Publication No. 2018/221685). In addition, mutants of the Cas protein that have lost part or all of their nuclease activity (nCas, dCas) are known, and when these mutants are used, they can be fused with other proteins with nuclease activity (such as FokI) and act on the target as a fusion protein to cleave the target. Note that in the CRISPR-Cas3 system, a form where a fusion protein of Cascade and another nuclease (FokI) is used instead of Cas3 is also known (International Publication No. 2013/098244). When such an artificial nuclease is used, the site-specific nuclease system described herein is constructed as a "guide RNA-guided artificial nuclease system" (a form of guide RNA-guided nuclease system).

In addition, the Cas protein may have other mutations or other peptides added to it, as long as the purpose described herein is not impaired. Other mutations include, for example, mutations to reduce or lose helicase activity, and other peptides to be added include, for example, signal sequences (such as nuclear translocation signal, mitochondrial translocation signal, and chloroplast translocation signal), labeled proteins (such as fluorescent proteins), and tags (such as His tags).

On the other hand, the artificial nucleases that constitute the artificial nuclease system are typically fusion proteins that contain a DNA-binding domain and a nuclease domain. The fusion protein component can be, for example, in the form of a protein, in the form of mRNA encoding the protein, or in the form of a vector expressing the protein.

The TALEN system is a fusion protein containing the DNA-binding domain and nuclease domain of transcription activator-like (TAL) effectors (for example, International Publication No. 2012/104729, International Publication No. 2011/072246, International Publication No. 2015/019672, and the like). In addition, the ZFN system is a fusion protein containing a DNA-binding domain and a nuclease domain, including a zinc finger array (for example, International Publication No. 2003/087341). In addition, the PPR system is a fusion protein containing a PPR (pentatricopeptide repeat) domain and a nuclease domain (Nakamura et al., Plant Cell Physiol 53: 1171-1179 (2012), International Publication No. 2014/175284). Examples of typical nuclease domains in these artificial nuclease systems include, but are not limited to, FokI, as long as they can cleave DNA. The nuclease domain may cleave the target DNA by forming a dimer, in which case two fusion proteins are used in one DNA cleavage. The nuclease domain may be bound to the C-terminal side of the DNA-binding domain or to the N-terminal side thereof (Beurdeley M, et al., Nat Commun. 2013; 4: 1762. doi:10.1038).

These fusion proteins are targeted to the target DNA sequence by a DNA-binding domain constructed by linking modules (peptides) that recognize specific bases (or specific nucleotide sequences), and cleave the target DNA by a nuclease domain fused to that DNA-binding domain. An appropriate spacer peptide may be introduced between the DNA-binding domain and the nuclease domain in the fusion protein. The fusion protein may be introduced with a mutation or added with other peptides as long as the purpose described herein is not impaired. The mutation includes, for example, mutations in the nuclease domain (such as FokI) to form heterodimers, and other peptides to be added include, for example, signal sequences (such as nuclear translocation signal, mitochondrial translocation signal, and chloroplast translocation signal), labeled proteins (such as fluorescent proteins), and tags (such as His tags).

The components of the site-specific nuclease system, when employed in the form of expression vectors, include one or more regulatory elements that are operatively bound to the DNA to be expressed. The DNA to be expressed may be modified to a nucleotide sequence suitable for expression in a host cell.

Here, "operably bound" means that the above DNA is expressibly bound to a regulatory element. The "regulatory elements" include promoters, enhancers, internal ribosomal entry sites (IRES), and other expression control elements (such as transcription termination signals and polyadenylation signals). The regulatory elements may be directed to, for example, the constitutive expression of DNA in a variety of host cells or to the expression of DNA only in specific cells, tissues, or organs, depending on the purpose. In addition, they may also be directed to the expression of DNA only at a specific time or to artificially inducible DNA expression. The promoters include, for example, polIII promoter (such as U6 and H1 promoters), polII promoter (such as Rous sarcoma virus (RSV) LTR promoter of retroviruses, cytomegalovirus (CMV) promoter, SV40 promoter, dihydrofolate reductase promoter, β-actin promoter, phosphoglycerol kinase (PGK) promoter, and EF1α promoter), polI promoter, or combinations thereof. Those skilled in the art can select an appropriate expression vector depending on the type and the like of cell to be introduced.

The "donor DNA" used herein contains a nucleotide sequence in which a 5'-side homology arm sequence, a donor sequence, and a 3'-side homology arm sequence are arranged in this order from the 5'-side. The donor DNA may contain other nucleotide sequences as long as the purpose of the method described herein is not impaired.

When the design is based on the sense strand of the genome-editing target region, the 5'-side homology arm sequence is the nucleotide sequence that has identity with the 5' outer nucleotide sequence of the sense strand of the genome-editing target region, and the 3'-side homology arm sequence is the nucleotide sequence that has identity with the 3' outer nucleotide sequence of the sense strand of the genome-editing target region. On the other hand, when the design is based on the antisense strand of the genome target region, the 5'-side homology arm sequence is the nucleotide sequence that has identity with the 5' outer nucleotide sequence of the antisense strand of the genome-editing target region, and the 3'-side homology arm sequence is the nucleotide sequence that has identity with the 3' outer nucleotide sequence of the antisense strand of the genome-editing target region.

The nucleotide sequence identity of the homology arm sequence of the donor DNA and the corresponding sequence on the genomic DNA is, for example, 80%or more, preferably 85%or more, more preferably 90%or more, further preferably 95%or more, still further preferably 97%or more, still further preferably 98%or more, still further preferably 99%or more, and still further preferably 100%. The sequence identity can be calculated using BLAST or other methods (for example, default parameters).

There are no particular restrictions on the length of the homology arm sequence, but for example, it can be 30 bases long or more (for example, 50 bases long or more, and 100 bases long or more), and 2000 bases long or less (for example, 1000 bases long or less, and 500 bases long or less).

By the method described herein, a donor sequence is inserted between the genomic sequences corresponding to the homology arms on both sides. The desired DNA sequence can be selected as the donor sequence, and for example, a mutated genomic sequence or foreign gene sequence can be used. If there is a nucleotide sequence in the desired DNA that is to be removed a posteriori, recognition sequences of the recombinase (such as loxP sequence and FRT sequence) can be added to both ends of the sequence, for example. The nucleotide sequence flanked by the recognition sequences of the recombinase can be removed by the action of the corresponding recombinase (such as Cre recombinase or FLP recombinase) or activity-inducing recombinase (such as CreERT2). In addition, for example, a selection marker sequence (such as fluorescent protein, drug-resistant gene, or negative selection marker gene) can also be incorporated into the desired DNA to confirm the success of the knock-in or other purposes and the like. When a gene is included in the desired DNA, a promoter or other control sequence can be operably linked to it. Examples of promoters include constitutive promoters, tissue-specific promoters, time-specific promoters, inducible promoters, and CMV promoters. Other elements, such as terminator sequences, can be added as appropriate.

There are no particular restrictions on the length of the donor sequence as long as it does not interfere with the construction of the donor DNA. For example, it is 5 bases long or more (for example, 10 bases long or more, and 30 bases long or more), and 30000 bases long or less (for example, 10000 bases long or less, 5000 bases long or less, and 3000 bases long or less).

The form of donor DNA is preferably a double-stranded cyclic DNA. A double-stranded cyclic DNA can be produced by a known technique (such as PCR, restriction enzyme cleavage, or DNA linkage technology) or can be purchased.

In the method described herein, for the purpose of producing knock-in of the donor sequence by combinational repair of non-homologous end joining and homologous recombination, the site-specific nuclease system targets and cleaves the sequences (i) to (iii) of group A or group B below.
Group A:
   (i) 5'-side homology arm sequence
   (ii) the sequence of the 5'-side homology arm-corresponding genomic region (hereinafter referred to as the "5'-side homology arm-corresponding genomic sequence")
   (iii) the sequence 3'-side downstream from the cleavage site of the sequence (ii) by the site-specific nuclease system (hereinafter referred to as the "3'-side downstream genomic sequence")
Group B
   (i) 3'-side homology arm sequence
   (ii) the sequence of the 3'-side homology arm-corresponding genomic region (hereinafter referred to as the "3'-side homology arm-corresponding genomic sequence")
   (iii) the sequence in the genome-editing target region that is 5'-side upstream from the cleavage site of the sequence (ii) by the site-specific nuclease system (hereinafter referred to as the "5'-side upstream genomic sequence")

Here, the "5'-side homology arm sequence" and the "5'-side homology arm-corresponding genomic sequence" of group A have homologous nucleotide sequences, so that once a site-specific nuclease system that targets one of them is constructed, the system will also target the other. In other words, the same molecules that constitute the site-specific nuclease system will be targeted. On the other hand, the "3'-side downstream genomic sequence" is targeted by other molecules that constitute the site-specific nuclease system. The "3'-side downstream genomic sequence" is specifically the sequence 3'-side downstream of the cleavage site in the 5'-side homology arm-corresponding genomic sequence, the sequence of the genomic intermediate region (if present), and the 3'-side homology arm-corresponding genomic sequence.

Similarly, for the "3'-side homology arm sequence" and the "3'-side homology arm-corresponding genomic sequence" of group B, the same molecules that constitute the site-specific nuclease system are targeted, and the "5'-side upstream genomic sequence" is targeted by other molecules that constitute the site-specific nuclease system. The "5'-side upstream genomic sequence" is specifically the 5'-side homology arm-corresponding genomic sequence, the sequence of the genomic intermediate region (if present), and the sequence 5'-side upstream of the cleavage site in the 3'-side homology arm-corresponding genomic sequence.

For example, if the site-specific nuclease system is a CRISPR/Cas system, the CRISPR/Cas system of (a) or (b) below can be used in the method described herein.
(a) A CRISPR/Cas system containing a combination of a guide RNA targeting the "5'-side homology arm sequence/5'-side homology arm-corresponding genomic sequence" (hereinafter referred to as "guide RNA 1") and a guide RNA targeting the "3'-side downstream genomic sequence" (hereinafter referred to as "guide RNA 2")
(b) A CRISPR/Cas system containing a combination of a guide RNA targeting the "3'-side homology arm sequence/3'-side homology arm-corresponding genomic sequence" (hereinafter referred to as "guide RNA 1‴) and a guide RNA targeting the "5'-side upstream genomic sequence" (hereinafter referred to as "guide RNA 2'")

In addition, if the site-specific nuclease system is an artificial nuclease system, the following artificial nuclease systems (a) or (b) can be used in the method described herein.
(a) An artificial nuclease system containing a combination of a fusion protein containing a DNA-binding domain and nuclease domain that target the "5'-side homology arm sequence/5'-side homology arm-corresponding genomic sequence" (hereinafter referred to as the "fusion protein 1") and a fusion protein containing a DNA-binding domain and nuclease domain that target the "3'-side downstream genomic sequence" (hereinafter referred to as the "fusion protein 2")
(b) An artificial nuclease system containing a combination of a fusion protein containing a DNA-binding domain and nuclease domain that target the "3'-side homology arm sequence/3'-side homology arm-corresponding genomic sequence" (hereinafter referred to as the "fusion protein 1'") and a fusion protein containing a DNA-binding domain and nuclease domain that target the "5'-side upstream genomic sequence" (hereinafter referred to as the "fusion protein 2'")

The artificial nuclease may cleave the target by dimerization of its nuclease domain (such as FokI), in which case two fusion proteins are used to produce a single cleavage. The methods for designing such fusion proteins are known (for example, International Publication No. 2011/072246 and the like).

The site-specific nuclease that targets and cleaves the "3'-side downstream genomic sequence" preferably targets the boundary region (nucleotide sequence including the border) between the genomic intermediate region and the 3'-side homology arm-corresponding genomic region in the genome-editing target region (see Fig. 4A). Since the donor DNA does not have the sequence of the genomic intermediate region, the site-specific nuclease in this form cannot target the donor DNA but specifically targets the "3'-side downstream genomic sequence." Note that if there is no genomic intermediate region in the genome-editing target region, it is preferable that the site-specific nuclease targets the boundary region between the 5'-side homology arm-corresponding genomic region and the 3'-side homology arm-corresponding genomic region in the genome-editing target region (see Figs. 2A and 3A). Since the donor DNA has a donor sequence between the 5'-side homology arm sequence and the 3'-side homology arm sequence, the site-specific nuclease in this form cannot target the donor DNA but specifically targets and cleaves the "3'-side downstream genomic sequence."

In the same principle, the site-specific nuclease that targets and cleaves the "5'-side upstream genomic sequence" preferably targets the boundary region between the 5'-side homology arm-corresponding genomic region and the genomic intermediate region in the genome-editing target region. Since the donor DNA does not have the sequence of the genomic intermediate region, the site-specific nuclease in this form cannot target the donor DNA but specifically targets the "5'-side upstream genomic sequence." Note that if there is no genomic intermediate region in the genome-editing target region, it is preferable that the site-specific nuclease targets the boundary region between the 5'-side homology arm-corresponding genomic region and the 3'-side homology arm-corresponding genomic region in the genome-editing target region. Since the donor DNA has a donor sequence between the 5'-side homology arm sequence and the 3'-side homology arm sequence, the site-specific nuclease in this form cannot target the donor DNA but specifically targets and cleaves the "5'-side upstream genomic sequence."

In the site-specific nuclease system described herein, the homology arm-corresponding genomic region and genomic intermediate region can be set up in the following way. For example, by setting a target DNA sequence of the targeting molecule (such as guide RNA 2 or fusion protein 2) on the genomic DNA and setting a 3'-side homology arm for the nucleotide sequence downstream from a specific base in the target DNA sequence (for example, in the case of guide RNA 2 of the CRISPR-Cas9 system, the 3'-side base of the cleavage site that exists in the target DNA sequence) so that the desired DNA cleavage occurs, the boundary region between the genomic intermediate region and the 3'-side homology arm-corresponding genomic region can be targeted by the targeting molecule. Similarly, by setting a target DNA sequence of the targeting molecule (such as guide RNA 2' or fusion protein 2') on the genomic DNA and setting a 5'-side homology arm for the nucleotide sequence upstream from a specific base in the target DNA sequence (for example, in the case of guide RNA 2' of the CRISPR-Cas9 system, the 5'-side base of the cleavage site that exists in the target DNA sequence) so that the desired DNA cleavage occurs, the boundary region between the 5'-side homology arm-corresponding genomic region and the genomic intermediate region can be targeted by the targeting molecule. Note that if there is no genomic intermediate region in the genome-editing target region, by setting a target DNA sequence of the targeting molecule (such as guide RNA 2, guide RNA 2', fusion protein 2, or fusion protein 2') on the genomic DNA and setting a 5'-side homology arm for the nucleotide sequence 5'-side upstream from a specific base in the target DNA sequence so that the desired DNA cleavage occurs, and by setting a 3'-side homology arm for the 3'-side downstream nucleotide sequence so that it will be contiguous to the nucleotide sequence for which the 5'-side homology arm has been set, the boundary region between the 5'-side homology arm-corresponding genomic region and the 3'-side homology arm-corresponding genomic region can be targeted by the targeting molecule.

If the site-specific nuclease system described herein is a guide RNA-guided nuclease system, the site of double-strand break is usually inside the target DNA sequence or near the outside of the target DNA sequence, and if the site-specific nuclease system is an artificial nuclease system, the site of double-strand break is usually outside the target DNA sequence.

When a site-specific nuclease system targets a boundary region, the boundary region is usually within 50 bases, preferably within 40 bases (for example, within 30 bases, and within 20 bases) from the above boundary in the design of the system. In this case, the site of double-strand break is usually within 100 bases, preferably within 50 bases (for example, within 40 bases, 30 bases, 20 bases, 10 bases, 5 bases, and 3 bases) from the above boundary.

If a genomic intermediate region exists in the genome-editing target region, the site-specific nuclease can also target the genomic intermediate region (or the boundary region between the genomic intermediate region and a homology arm-corresponding genomic region where non-homologous end joining occurs). Since the donor DNA does not have the sequence of the genomic intermediate region, the site-specific nuclease in this form cannot target the donor DNA but specifically targets the genomic intermediate region (or the boundary region between the genomic intermediate region and a homology arm-corresponding genomic region where non-homologous end joining occurs) .

For example, by setting a targeting molecule (such as guide RNA 2 or fusion protein 2) and setting a 3'-side homology arm for the nucleotide sequence downstream of the target DNA sequence of the targeting molecule so that the desired DNA cleavage occurs, the genomic intermediate region can be targeted by the targeting molecule. Similarly, by setting a targeting molecule (such as guide RNA 2' or fusion protein 2') and setting a 5'-side homology arm for the nucleotide sequence upstream of the target DNA sequence of the targeting molecule so that the desired DNA cleavage occurs, the genomic intermediate region can be targeted by the targeting molecule.

Note that if a long genomic intermediate region remains uncleaved with respect to the homology arm-corresponding genomic region on the side that contributes to homologous recombination after cleavage by the site-specific nuclease, the efficiency of homologous recombination may be reduced, and therefore, it is preferable to set the target DNA sequence on the genomic intermediate region so that the remaining genomic intermediate region is shortened by cleavage by the site-specific nuclease.

There are no particular restrictions on the cells into which the site-specific nuclease system is introduced, as long as they are cells in which the site-specific nuclease system functions. The cells can be eukaryotic cells such as animal cells, plant cells, algal cells, and fungal cells, or prokaryotic cells such as bacteria and archaea. The term "cell" includes, for example, a cell that constitutes an individual, a cell that constitutes an organ or tissue removed from an individual, or a cultured cell derived from the tissues of an individual.

The animal cells include, for example, blood cells, hematopoietic stem cells and progenitor cells, non-human fertilized eggs, non-human embryonic cells of embryos at various stages (such as 1-cell stage embryos, 2-cell stage embryos, 4-cell stage embryos, 8-cell stage embryos, 16-cell stage embryos, and mulberry stage embryos), fibroblasts, epithelial cells, vascular endothelial cells, nerve cells, hepatocytes, bone cells, keratin-producing cells, muscle cells, epidermal cells, endocrine cells, ES cells, iPS cells, tissue stem cells, cancer cells, and the like.

Animals from which animal cells are derived include, for example, mammals (such as humans, monkeys, mice, rats, guinea pigs, hamsters, pigs, cattle, goats, sheep, dogs, cats, and rabbits), fish (such as tiger pufferfish, tuna, red seabream, mackerel, zebrafish, goldfish, and killifish), birds (such as chickens, quails, turkeys, domestic ducks, geese, onagadori, bantams, pigeons, ostriches, pheasants, and guinea fowls), reptiles (such as snakes and lizards), amphibians (such as frogs and salamanders), and insects (such as flies and silkworms). Disclosed herein and not part of the invention, when producing knock-in non-human animals as model animals, the animals are preferably rodents such as mice, rats, guinea pigs, and hamsters, and particularly preferably mice or rats.

Plants from which plant cells are derived include, for example, cereals, oil crops, forage crops, fruits, and vegetables. Specific plants include, for example, thale cress, tomato, soybean, rice, wheat, barley, corn, rapeseed, tobacco, banana, peanut, sunflower, potato, cotton, and carnation.

Described herein are non-human germ cells and pluripotent stem cells for the production of knock-in non-human animals. For example, a non-human oocyte prepared by introducing a site-specific nuclease system into an oocyte can be transplanted into the oviduct or uterus of a pseudopregnant animal to obtain a litter in which the donor sequence has been inserted into the genome. From the obtained individuals, their offspring or clones can also be obtained.

Here, if a non-human fertilized egg containing an expression cassette of the recombinant enzyme (such as Cre, CreERT2, or FLP) is used, and the desired gene flanked by the recognition sequences of the recombinant enzyme (such as loxP sequence and FRT sequence) is used as the donor sequence, and the donor sequence is inserted into the genome of the fertilized egg by the method described herein, a conditional knock-out animal can be efficiently produced. When a non-human fertilized egg that does not contain the expression cassette of the recombinant enzyme (such as Cre, CreERT2, or FLP) is used, the individual obtained (individual that retains the desired gene flanked by the recognition sequences of the recombinant enzyme) can be crossed with an individual expressing the recombinant enzyme to produce a conditional knock-out animal. Such a conditional knock-out technology is useful, for example, in the production of disease models.

On the other hand, in plants, it has long been known that their somatic cells have differentiation totipotency, and methods for regenerating plant bodies from plant cells have been established in various plants. Thus, for example, by introducing a site-specific nuclease system into a plant cell and regenerating a plant body from the resulting plant cell, a plant body with a donor sequence inserted into the genome can be obtained. From the obtained plant body, progeny, clones, or propagating materials (such as tubers, tuberous roots, and seeds) can also be obtained. As the methods for obtaining individuals by redifferentiation of plant tissues through tissue culture, it is possible to use the methods established in the art (Protocols for Plant Transformation, edited by Yutaka Tabei, Kagaku-Dojin Publishing Co., Inc., pp. 340-347 (2012)).

The method for introducing the site-specific nuclease system and donor DNA into cells is not limited and can be appropriately selected according to the type of target cell and the type of material (whether it is nucleic acid, protein, or the like). Examples thereof include, but are not limited to, electroporation, microinjection, DEAE-dextran treatment, lipofection, nanoparticle-mediated transfection, and virus-mediated nucleic acid delivery. Each molecule constituting the site-specific nuclease system can be introduced simultaneously or separately.

In cells into which the site-specific nuclease system and donor DNA have been introduced as described above, the donor sequence is inserted into the genome-editing target region on the genomic DNA via two DNA repair mechanisms, that is, combinational repair of non-homologous end joining and homologous recombination.

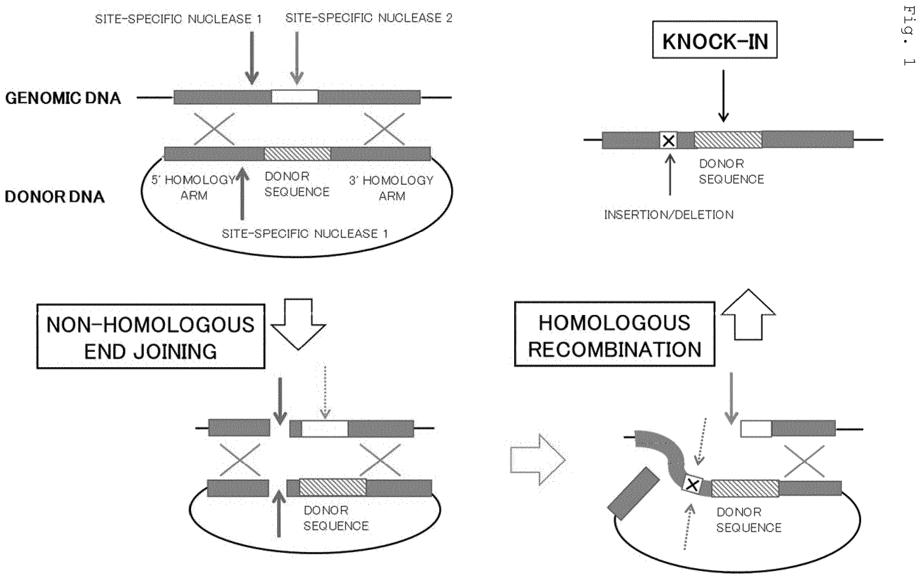

Fig. 1 shows the typical principle of the method described herein (as an example, when the 5' homology arm-corresponding genomic region and the genomic intermediate region are the targets of the site-specific nuclease system). First, under the action of the site-specific nuclease system, one of the cleavage ends created in the 5' homology arm-corresponding genomic region on the genomic DNA (the side not containing the genomic intermediate region) and one of the cleavage ends in the 5' homology arm on the donor DNA (the side containing the donor sequence) are joined by non-homologous end joining repair. In this repair, if a base mutation (such as addition or deletion) occurs in the cleavage site, it prevents the site-specific nuclease system from targeting it, and re-cleavage no longer occurs. Next, the repair via non-homologous end joining induces repair via homologous recombination between the 3' homology arm in the donor DNA moored to the genomic DNA and the 3' homology arm-corresponding genomic region near the cleavage end of the genomic intermediate region. As a result, the donor sequence is inserted into the genomic DNA.

The method described herein combines the advantage of "high frequency" of non-homologous end joining and the advantage of "high accuracy" of homologous recombination, allowing insertion of donor sequences with high efficiency and accuracy in genome-editing target regions on genomic DNA.

The present invention also provides use of a kit or composition in the method of the invention, i.e. for producing a cell in which a donor sequence is inserted into a genome-editing target region on a genomic DNA, comprising the above-mentioned site-specific nuclease system and donor DNA.

In the kit, the site-specific nuclease system and the donor DNA may be separate or integrated. Each component of the site-specific nuclease system may also be separate or integrated. The standard samples constituting the kit described herein may also contain additional components as necessary. Examples of the additional components include, but are not limited to, base agents, carriers, solvents, dispersants, emulsifiers, buffers, stabilizers, excipients, binders, disintegrants, lubricants, thickeners, humectants, colorants, flavors, chelating agents, and the like. The kit may further include additional elements. Examples of the additional elements include, but are not limited to, dilution buffers, reconstitution solutions, wash buffers, nucleic acid transfer reagents, protein transfer reagents, and control reagents (such as control guide RNA). The kit may include instructions for use in carrying out the method described herein.

The composition described herein contains the site-specific nuclease system and the donor DNA as an integral part. The composition described herein may further contain additional components as necessary. Examples of the additional components include, but are not limited to, base agents, carriers, solvents, dispersants, emulsifiers, buffers, stabilizers, excipients, binders, disintegrants, lubricants, thickeners, humectants, colorants, flavors, chelating agents, and the like. The composition described herein can also be a standard sample constituting the kit described herein.

### [Examples]

### [Example 1] Genome editing test 1 (wild-type mouse, Kcnab1 gene, microinjection)

The sequence around the termination codon of exon 14 of the mouse Kcnab1 (Potassium Voltage-Gated Channel Subfamily A Member Regulatory Beta Subunit 1) gene was used as the target DNA sequence for genome editing, and a donor sequence of 3983 bases was incorporated (Fig. 2A).

### (1) Preparation of Cas9 mRNA

A linearized plasmid containing a sequence with a poly-A tail added downstream of the Cas9 coding sequence (T7-NLS-hCas9-polyA, RIKEN BRC #RDB13130) was used as template DNA, synthesized using an in vitro transcription kit (MEGAshortscript T7 Transcription Kit, manufactured by Life Technologies), and purified using a purification kit (MEGAClear kit, manufactured by Life Technologies).

### (2) Preparation of gRNA

The design of gRNA was performed using the design support tool (http://crispor.tefor.net/). The gRNA1 (GTATAAATGACTGCTTAATGTGG/SEQ ID NO: 19, underlined is PAM), which cleaves both the target DNA sequence of one genome editing and the 5' homology arm of the donor DNA, and the gRNA2 (AAAGGACTATAGATCATAAGG/ SEQ ID NO: 20, underlined is PAM), which cleaves the target DNA sequence of the other genome editing, were synthesized using the gRNA in vitro synthesis kit (GeneArt (registered trademark) Precision gRNA Synthesis Kit, manufactured by Life Technologies).

Note that the gRNA2 is set across the genomic sequence corresponding to the 5' homology arm and the genomic sequence corresponding to the 3' homology arm on the genomic DNA. In the donor DNA, the gRNA2 cannot target the donor DNA because the donor sequence exists between both homology sequences.

### (3) Preparation of donor DNA

A double-stranded cyclic DNA as the donor DNA was produced by a genetic engineering method such as PCR, restriction enzyme cleavage, or DNA linkage technology. The homology arms on both sides were prepared by using the C57BL/6J strain mouse genome as an amplification template. The P2A sequence was prepared by using primer dimer formation in PCR. ERT2 and iCre sequences were prepared by using existing plasmids (Addgene #13777 and #51904) as amplification templates. The obtained fragments were linked to the pGEM-T vector (manufactured by Promega) to produce the donor vectors "intron-Ctgf-P2A-ERT2iCreERT2-pGEM" and "Kcnab1-P2A-ERT2iCreERT2-stop-3UTR2polyA-pGEM."

### (4) Introduction into fertilized eggs and genotyping

C57BL/6J Jcl pronuclear stage frozen embryos (manufactured by CLEA Japan, Inc.) were thawed and cultured at constant temperature in KSOM medium (manufactured by Arc Resources). The gRNA (25 ng/µL), Cas9 mRNA (50 ng/µL), and donor DNA (1 ng/µL) prepared in (2) above were microinjected into the male pronuclei in the embryos using a micromanipulator (manufactured by Narishige). The embryos were cultured at constant temperature, and the next day two-cell stage embryos were transplanted in a trans-oviduct manner into pseudopregnant female mice. Genomic DNA was extracted from the tails of born mice (F0 mice) using a kit (KAPA Express Extract DNA Extraction Kit, manufactured by Kapa Biosystems), and PCR screening was performed using primer sets designed for the outer side of the homology arm equivalent sequence and the inner side of the insertion sequence ("Upstream Connection Confirmation" and "Downstream Connection Confirmation" in Table 1) or the inner sides of the insertion sequences ("Insert Confirmation" in Table 1). Genotyping was performed by sequencing of the obtained PCR fragments. Primer sets are shown in Table 1.

**[Table 1]**

| Gene | Primer Name | 5'-Primer-3' | Usage |
|---|---|---|---|
| Kcnab1 | iCre-gp-fw (F2) | ACTCTGACAGATGCCAGGAC/SEQ ID NO: 1 | Insert Confirmation |
| | iCre-gp-rv (R2) | ATTCTTTCTGACCCGGCAGA/SEQ ID NO: 2 | |
| | Kcnab1-UA-fw3 (F1) | GCGTGCACCCGAGTGTGGTACTTAAG/SEQ ID NO: 3 | Upstream Connection Confirmation |
| | P2A-C-rv (R1) | TCCAGGGTTCTCCTCCACGTCTCC/SEQ ID NO: 4 | |
| | ERT2-C-seq-fw (F2) | CTGTCCAGCACCCTGAAGTC/SEQ ID NO: 5 | Downstream Connection Confirmation |
| | Kenab1-ds-rv (R3) | GGCACAACTTCTGTTGAGGC/SEQ ID NO: 6 | |
| Ctgf | iCre-gp-fw (F2) | ACTCTGACAGATGCCAGGAC/SEQ ID NO: 7 | Insert Confirmation |
| | iCre-gp-rv (R2) | ATTCTTTCTGACCCGGCAGA/SEQ ID NO: 8 | |
| | Ctgf-us-fw (F1) | AGCTGTCTGGCTAGAATGAG/SEQ ID NO: 9 | Upstream Connection Confirmation |
| | P2A-C-rv (R1) | TCCAGGGTTCTCCTCCACGTCTCC/SEQ ID NO: 10 | |
| | ERT2-C-seq-fw (F2) | CTGTCCAGCACCCTGAAGTC/SEQ ID NO: 11 | Downstream Connection Confirmation |
| | Ctgf-ds-rv (R3) | AGGCTTGGCTGTTCATTCTTGTA/SEQ ID NO: 12 | |
| Tp53 | ECFP-F (F2) | GGCAAGCTGACCCTGAAGTT/SEQ ID NO: 13 | Insert Confirmation |
| | ECFP-R (R2) | TCTCGTTGGGGTCTTTGCTC/SEQ ID NO: 14 | |
| | combi-ECFP-F (F1) | ACCTCCACACTTGTCTTGGC/SEQ ID NO: 15 | Upstream Connection Confirmation |
| | CAGGS-CAG-R (R1) | CCGCCTCGCCATAAAAGGAA/SEQ ID NO: 16 | |
| | ECFP-F (F2) | GGCAAGCTGACCCTGAAGTT/SEQ ID NO: 17 | Downstream Connection Confirmation |
| | combi-ECFP-R (R3) | CACCGTCACCATCAGAGCAA/SEQ ID NO: 18 | |

### (5) Results

The results of the present Example are shown in Table 2. In 33.3% of the mice born, F0 mice were obtained that had alleles with the ERT2-iCre-ERT2 sequence inserted into the target DNA sequence for genome editing (that is, mice for which the desired genome editing was successful) (Table 2, "Combi" for "Kcnab1-ERT2iCreERT2"). Experiments using different methods aimed at inserting the same sequence did not yield mice with successful genome editing (Table 2, "lssDNA" for "Kcnab1-ERT2iCreERT2"). Note that the method using lssDNA was based on the method reported in the literature (Miyasaka, Y. et al. BMC Genomics. 2018; 19(1): 318).

**[Table 2]**

| Gene Name | Method | KI Size (bp) | Introduction method | Cas9 Type | Total Number of Embryos Introduced | Total Number of Litters Born | Number of KI Individuals | Ratio of KI Individuals |
|---|---|---|---|---|---|---|---|---|
| Kcnab1-ERT2iCreERT2 | LssDNA | 3345 | EP/Inj. | mRNA | 171 | 24 | 0 | 0 % |
| | Combi | 3983 | Injection | mRNA | 148 | 9 | 3 | 33.3 % |
| Ctgf-ERT2iCreERT2 | LssDNA | 3345 | EP/Inj. | mRNA | 178 | 18 | 0 | 0 % |
| | Combi | 3556 | Injection | mRNA | 151 | 6 | 1 | 16.7 % |
| Tp53-CFP | 3H20P | 4513 | Injection | mRNA/Protein | 618 | 34 | 0 | 0 % |
| | Combi | 4513 | Injection | Protein | 174 | 6 | 1 | 16.6 % |

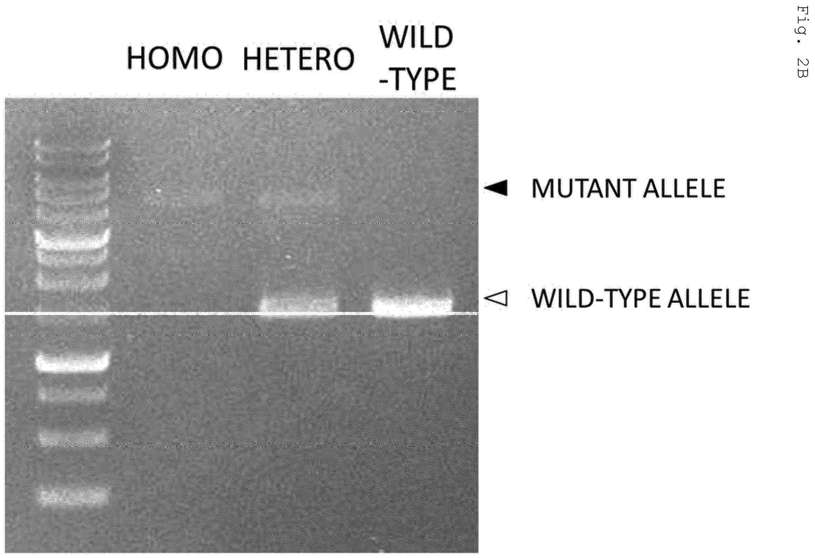

In addition, genotyping of the F1 mice obtained by crossing these F0 mice with C57BL/6J Jcl mice also confirmed germline transmission of alleles subjected to the desired genome editing (Fig. 2B). In addition, to confirm that the inserted iCre had the desired function as a recombinase, F1 mice were crossed with Ai14 mice expressing the tdTomato reporter in a recombination-dependent manner (Jackson Laboratory Stock No: 007914). Specifically, after tamoxifen was administered to the litters obtained from the crossing on the 2nd day of life, brain tissues were removed on the 5th day of life and vibratome sections were produced for observation, and as a result, tdTomato-positive cells were found in the expected region of the cerebral cortex.

### [Example 2] Genome editing test 2 (wild-type mouse, Ctgf gene, microinjection)

The sequence around the termination codon of exon 5 of the mouse Ctgf (connective tissue growth factor) gene was used as the target DNA sequence for genome editing, and a donor sequence of 3556 bases was incorporated (Fig. 3A). The preparation of Cas9 mRNA, preparation of gRNA, preparation of donor DNA, introduction into fertilized eggs, and genotyping were performed in the same manner as in Example 1. The gRNA sequences used are as follows, respectively. In addition, the primer sets used are also shown in Table 1.
gRNA1:GACATAGGGCTAGTCTACAAAGG/SEQ ID NO: 21, underlined is PAM
gRNA2:CGGAGACATGGCGTAAAGCCAGG/SEQ ID NO: 22, underlined is PAM

Note that the gRNA2 is set across the genomic sequence corresponding to the 5' homology arm and the genomic sequence corresponding to the 3' homology arm on the genomic DNA. In the donor DNA, the gRNA2 cannot target the donor DNA because the donor sequence exists between both homology sequences.

The results of the present Example are shown in Table 2. In 16.7% of the mice born, F0 mice were obtained that had alleles with the P2A-ERT2-iCre-ERT2 sequence inserted into the target DNA sequence for genome editing (that is, mice for which the desired genome editing was successful) (Table 2, "Combi" for "Ctgf-ERT2iCreERT2"). Experiments using different methods aimed at inserting the same sequence did not yield mice with successful genome editing (Table 2, "lssDNA" for "Ctgf-ERT2iCreERT2").

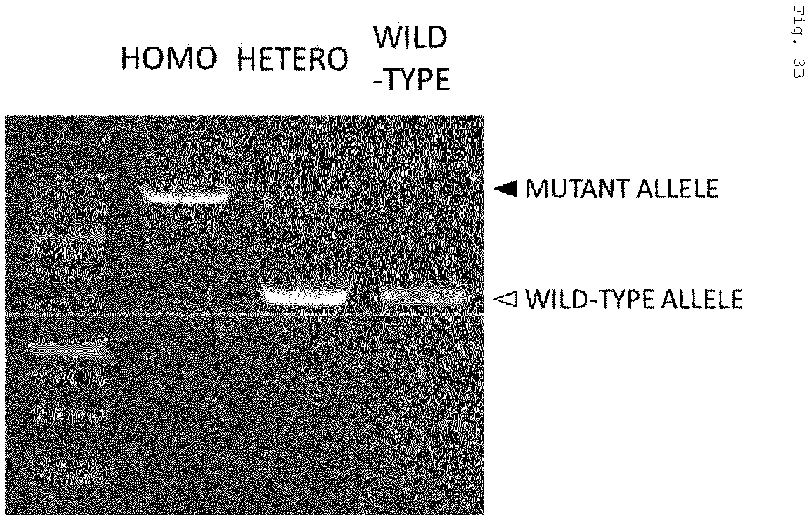

In addition, genotyping of the F1 mice obtained by crossing these F0 mice with C57BL/6J Jcl mice also confirmed germline transmission of alleles subjected to the desired genome editing (Fig. 3B).

In addition, to confirm that the inserted iCre had the desired function as a recombinase, F1 mice were crossed with Ai14 mice expressing the tdTomato reporter in a recombination-dependent manner (Jackson Laboratory Stock No: 007914). To be more specific, after tamoxifen was administered to the litters obtained from the crossing on the 2nd day of life, brain tissues were removed on the 5th day of life and vibratome sections were produced for observation. As a result, tdTomato-positive cells were found in the expected region of the cerebral cortex.

### [Example 3] Genome editing test 3 (wild-type rat, Tp53 gene, microinjection)

The sequences around exon 2 and exon 4 of the rat Tp53 (tumor protein p53) gene were used as the target DNA sequences for genome editing, and a donor sequence of 4513 bases was incorporated (Fig. 4A).

### (1) Preparation of a Cas9 protein-gRNA complex solution and donor DNA

The gRNA(CCCTGCCAGATAGTCCACCTTCT/SEQ ID NO: 23, underlined is PAM), which cleaves both the target DNA sequence of genome editing in exon2 and the 5' homology arm of the donor DNA, and the gRNA (CCACAGCGACAGGGTCACCTAAT/SEQ ID NO: 24, underlined is PAM), which cleave the target DNA sequence of genome editing in exon4, were used. The design of gRNA was performed using the design support tool (http://crispor.tefor.net/).

Note that the gRNA2 is set across the genomic sequence corresponding to the 3' homology arm and the genomic sequence located upstream thereof (the 5' end portion of exon4) on the genomic DNA. In the donor DNA, the gRNA2 cannot target the donor DNA because there is no sequence corresponding to the 5' end portion of exon4.

Mixed were 1.75 µL each of crRNA and tracrRNA (Alt-R (registered trademark) CRISPR/Cas9 System, manufactured by Integrated DNA Technologies) prepared to 100 µM and 1.5 µL of the binding buffer packed together. The mixture was heated at 95°C for 5 minutes and then cooled slowly to cause complementary binding, forming a gRNA. Mixed were 1250 ng of the above gRNA and 5000 ng of purified Cas9 protein (Alt-R (registered trademark) S. p. Cas9 Nuclease 3NLS, manufactured by Integrated DNA Technologies) to form a Cas9 protein/gRNA complex (ribonucleoprotein). The donor DNA selected was a region for setting 5'-side and 3'-side homology arms from exon2 and exon4 of rat Tp53 and their surrounding sequences, and these homology arms were used to design a nucleotide sequence flanking the CAG promoter and the fluorescent protein (CFP) gene. The plasmid containing the above donor DNA sequence was constructed using an artificial gene synthesis service (GeneArt (registered trademark) Gene Synthesis, manufactured by Life Technologies). The final concentrations in the microinjection solution are 100 ng/µL for Cas9 protein, 25 ng/µL for each gRNA, and 1 ng/µL for donor DNA.

### (2) Induction into fertilized eggs and genotyping

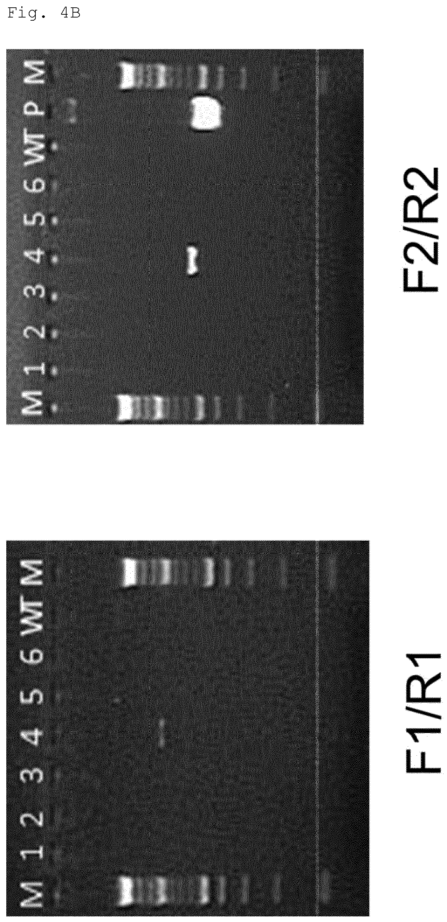

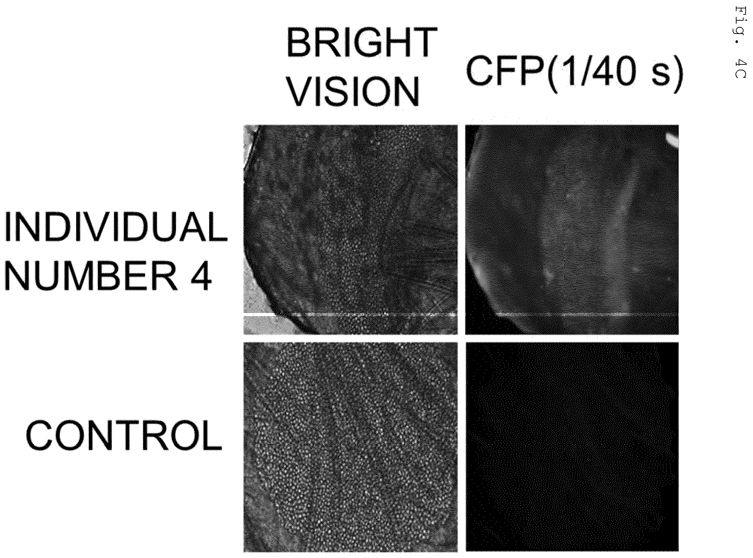

Jcl:SD female rats were superovulated by intraperitoneal injection of 150 U/kg of pregnant mare serum gonadotropin (manufactured by ASKA Pharmaceutical Co., Ltd.) followed by 75 U/kg of human chorionic gonadotropin (manufactured by ASKA Pharmaceutical Co., Ltd.) and mated with Jcl:SD male rats. On the following day, pronuclear stage embryos were harvested from the female rats for which mating was confirmed, and cultured at constant temperature in rat KSOM medium (manufactured by Arc Resources). An injection solution containing 100 ng/µL of Cas9 protein, 25 ng/µL of each gRNA, and 1 ng/µL of donor DNA prepared as described above was microinjected into the male pronuclei in the embryos using a micromanipulator (manufactured by Narishige). The embryos were cultured at constant temperature, and the two-cell stage embryos were transplanted in a trans-oviduct manner into pseudopregnant female rats. Genomic DNA was extracted from the tails of the born rats (F0 rats), and PCR screening was performed using primer sets designed for the outer side of the homology arm equivalent sequence and the inner side of the insertion sequence or the inner sides of the insertion sequence. Genotyping was performed by sequencing of the obtained PCR fragments. Primer sets are shown in Table 1. Deletion was observed in the upstream junction (Fig. 4B). In addition, although the downstream junction was undetectable by PCR, observation of the tissue fragments with a fluorescence microscope showed fluorescence (Fig. 4C).

### (3) Results

The results of the present Example are shown in Table 2. In 16.6% of the rats born, F0 rats were obtained that had alleles with the CFP sequence inserted into the target DNA sequence of genome editing (Table 2, "Combi" for "Tp53-CFP"). Experiments using different methods aimed at inserting the same sequence did not yield rats with successful genome editing (Table 2, "3H2OP" for "Tp53-CFP"). Note that the 3H3OP method was based on the method reported in the literature (Yoshimi, K. et al, Nat Commun. 2016; 7: 10431).

### [Industrial Applicability]

As described herein, cells and organisms in which donor sequences are inserted on the genome can be produced with high efficiency and accuracy. The present disclosure can be used, for example, in the production of pharmaceuticals, agricultural crops, processed foods, livestock products, marine products, industrial products, and laboratory animals, as well as in basic research in the field of life sciences.

### [Sequence Listing Free Text]

SEQ ID Nos: 1 to 19
   <223> Primer sequence
SEQ ID Nos: 20 to 24
   <223> Guide RNA sequence

## Claims

1. An *in vitro* method for producing a cell in which a donor sequence is inserted into a genome-editing target region on a genomic DNA, comprising the step of:
introducing a site-specific nuclease system and donor DNA into a cell, wherein
the donor DNA is a DNA containing a nucleotide sequence in which a 5'-side homology arm sequence, a donor sequence, and a 3'-side homology arm sequence are arranged in this order from a 5'-side,
the site-specific nuclease system is a system that targets and cleaves sequences (i) to (iii) of group A or group B below, and the sequences (i) and (ii) are targeted by the same molecules constituting the site-specific nuclease system, and the sequence (iii) is targeted by other molecules constituting the site-specific nuclease system:
Group A:
(i) the 5'-side homology arm sequence
(ii) a sequence in the genome-editing target region corresponding to the sequence (i)
(iii) a sequence in the genome-editing target region between a sequence corresponding to the 5'-side homology arm sequence and a sequence corresponding to the 3'-side homology arm sequence; and
Group B
(i) the 3'-side homology arm sequence
(ii) a sequence in the genome-editing target region corresponding to the sequence (i)
(iii) a sequence in the genome-editing target region between a sequence corresponding to the 5'-side homology arm sequence and a sequence corresponding to the 3'-side homology arm sequence,
wherein the site-specific nuclease system is:
a CRISPR/Cas system containing a combination of a guide RNA that targets the sequences (i) and (ii) and a guide RNA that targets the sequence (iii), or
an artificial nuclease system containing a combination of: a fusion protein that contains a DNA-binding domain that targets the sequences (i) and (ii) and a nuclease domain, and a fusion protein that contains a DNA-binding domain that targets the sequence (iii) and a nuclease domain,
wherein the method integrates the donor nucleic acid by the combinational repair of non-homologous end joining of the cleaved sequences (i) and (ii) and homologous recombination of the non-cleaved homology arm with the genomic sequence, wherein the method is not a process for modifying the germline genetic identity of human beings.

2. Use of a kit or composition in the method of claim 1, wherein the kit or composition comprises:
a site-specific nuclease system and donor DNA, wherein
the donor DNA is a DNA containing a nucleotide sequence in which a 5'-side homology arm sequence, a donor sequence, and a 3'-side homology arm sequence are arranged in this order from a 5'-side,
the site-specific nuclease system is a system that targets and cleaves sequences (i) to (iii) of group A or group B below, and the sequences (i) and (ii) are targeted by the same molecules constituting the site-specific nuclease system, and the sequence (iii) is targeted by other molecules constituting the site-specific nuclease system,
Group A:
(i) the 5'-side homology arm sequence
(ii) a sequence in the genome-editing target region corresponding to the sequence (i)
(iii) a sequence in the genome-editing target region between a sequence corresponding to the 5'-side homology arm sequence and a sequence corresponding to the 3'-side homology arm sequence
Group B
(i) the 3'-side homology arm sequence
(ii) a sequence in the genome-editing target region corresponding to the sequence (i)
(iii) a sequence in the genome-editing target region between a sequence corresponding to the 5'-side homology arm sequence and a sequence corresponding to the 3'-side homology arm sequence
wherein the site-specific nuclease system is:
a CRISPR/Cas system containing a combination of a guide RNA that targets the sequences (i) and (ii) and a guide RNA that targets the sequence (iii), or
an artificial nuclease system containing a combination of: a fusion protein that contains a DNA-binding domain that targets the sequences (i) and (ii) and a nuclease domain, and a fusion protein that contains a DNA-binding domain that targets the sequence (iii) and a nuclease domain.

## Patentansprüche

1. In-vitro-Verfahren zum Erzeugen einer Zelle, in dem eine Donor-Sequenz in eine Genomeditierungs-Zielregion an einer genomischen DNA eingefügt ist, umfassend den folgenden Schritt:
Einführen eines standortspezifischen Nukleasesystems und von Donor-DNA in eine Zelle, wobei
die Donor-DNA eine DNA ist, die eine Nukleotidsequenz enthält, in der eine 5'-seitige Homologiearmsequenz, eine Donor-Sequenz und eine 3'-seitige Homologiearmsequenz in dieser Reihenfolge von einer 5'-Seite angeordnet sind,
das standortspezifische Nukleasesystem ein System ist, das Sequenzen (i) bis (iii) von gruppe A oder Gruppe B unten anzielt und spaltet, und die Sequenzen (i) und (ii) werden durch die gleichen Moleküle angezielt, die das standortspezifische Nukleasesystem bilden, und die Sequenz (iii) wird durch andere Moleküle angezielt, die das standortspezifische Nukleasesystem bilden:
Gruppe A:
(i) die 5'-seitige Homologiearmsequenz
(ii) eine Sequenz in der Genomeditierungs-Zielregion, die der Sequenz (i) entspricht
(iii) eine Sequenz in der Genomeditierungs-Zielregion zwischen einer Sequenz, die der 5'-seitigen Homologiearmsequenz entspricht, und einer Sequenz, die der 3'-seitigen Homologiearmsequenz entspricht; und
Gruppe B
(i) die 3'-seitige Homologiearmsequenz
(ii) eine Sequenz in der Genomeditierungs-Zielregion, die der Sequenz (i) entspricht
(iii) eine Sequenz in der Genomeditierungs-Zielregion zwischen einer Sequenz, die der 5'-seitigen Homologiearmsequenz entspricht, und einer Sequenz, die der 3'-seitigen Homologiearmsequenz entspricht,
wobei das standortspezifische Nukleasesystem Folgendes ist:
ein CRISPR/Cas-System, das eine Kombination aus einer Guide-RNA enthält, die die Sequenzen (i) und (ii) anzielt, und einer Guide-RNA, die die Sequenz (iii) anzielt, oder
ein künstliches Nukleasesystem, das eine Kombination aus Folgendem enthält: einem Fusionsprotein, das eine DNA-bindende Domäne enthält, die die Sequenzen (i) und (ii) und eine Nukleasedomäne anzielt, und einem Fusionsprotein, das eine DNA-bindende Domäne enthält, die die Sequenz (iii) und eine Nukleasedomäne anzielt,
wobei das Verfahren die Donor-Nukleinsäure durch die kombinatorische Reparatur von nicht-homologer Endverknüpfung der gespaltenen Sequenzen (i) und (ii) und homologer Rekombination des nicht-gespaltenen Homologiearms mit der genomischen Sequenz integriert, wobei das Verfahren kein Prozess zum Modifizieren der genetischen Identität der Keimbahn von Menschen ist.

2. Verwendung eines Kits oder einer Zusammensetzung in dem Verfahren nach Anspruch 1, wobei das Kit oder die Zusammensetzung Folgendes umfasst:
ein standortspezifisches Nukleasesystem und Donor-DNA, wobei
die Donor-DNA eine DNA ist, die eine Nukleotidsequenz enthält, in der eine 5'-seitige Homologiearmsequenz, eine Donor-Sequenz und eine 3'-seitige Homologiearmsequenz in dieser Reihenfolge von einer 5'-Seite angeordnet sind,
das standortspezifische Nukleasesystem ein System ist, das Sequenzen (i) bis (iii) von Gruppe A oder Gruppe B unten anzielt und spaltet, und die Sequenzen (i) und (ii) werden durch die gleichen Moleküle angezielt, die das standortspezifische Nukleasesystem bilden, und die Sequenz (iii) wird durch andere Moleküle angezielt, die das standortspezifische Nukleasesystem bilden,
Gruppe A:
(i) die 5'-seitige Homologiearmsequenz
(ii) eine Sequenz in der Genomeditierungs-Zielregion, die der Sequenz (i) entspricht
(iii) eine Sequenz in der Genomeditierungs-Zielregion zwischen einer Sequenz, die der 5'-seitigen Homologiearmsequenz entspricht, und einer Sequenz, die der 3'-seitigen Homologiearmsequenz entspricht
Gruppe B
(i) die 3'-seitige Homologiearmsequenz
(ii) eine Sequenz in der Genomeditierungs-Zielregion, die der Sequenz (i) entspricht
(iii) eine Sequenz in der Genomeditierungs-Zielregion zwischen einer Sequenz, die der 5'-seitigen Homologiearmsequenz entspricht, und einer Sequenz, die der 3'-seitigen Homologiearmsequenz entspricht,
wobei das standortspezifische Nukleasesystem Folgendes ist:
ein CRISPR/Cas-System, das eine Kombination aus einer Guide-RNA enthält, die die Sequenzen (i) und (ii) anzielt, und einer Guide-RNA, die die Sequenz (iii) anzielt, oder
ein künstliches Nukleasesystem, das eine Kombination aus Folgendem enthält: einem Fusionsprotein, das eine DNA-bindende Domäne enthält, die die Sequenzen (i) und (ii) und eine Nukleasedomäne anzielt, und einem Fusionsprotein, das eine DNA-bindende Domäne enthält, die die Sequenz (iii) und eine Nukleasedomäne anzielt,

## Revendications

1. Procédé *in vitro* pour produire une cellule dans laquelle une séquence donneuse est insérée dans une région cible d'édition génomique sur un ADN génomique, comprenant l'étape consistant à :
introduire un système de nucléase spécifique à un site et un ADN donneur dans une cellule, dans lequel
l'ADN donneur est un ADN contenant une séquence nucléotidique dans laquelle une séquence de bras d'homologie côté 5', une séquence donneuse, et une séquence de bras d'homologie côté 3' sont agencées dans cet ordre à partir du côté 5',
le système de nucléase spécifique à un site est un système qui cible et clive les séquences (i) à (iii) du groupe A ou du groupe B ci-dessous, et les séquences (i) et (ii) sont ciblées par les mêmes molécules constituant le système de nucléase spécifique à un site, et la séquence (iii) est ciblée par d'autres molécules constituant le système de nucléase spécifique à un site :
Groupe A :
(i) la séquence de bras d'homologie côté 5'
(ii) une séquence dans la région cible d'édition génomique correspondant à la séquence (i)
(iii) une séquence dans la région cible d'édition génomique entre une séquence correspondant à la séquence de bras d'homologie côté 5' et une séquence correspondant à la séquence de bras d'homologie côté 3' ; et
Groupe B :
(i) la séquence de bras d'homologie côté 3'
(ii) une séquence dans la région cible d'édition génomique correspondant à la séquence (i)
(iii) une séquence dans la région cible d'édition génomique entre une séquence correspondant à la séquence de bras d'homologie côté 5' et une séquence correspondant à la séquence de bras d'homologie côté 3',
dans lequel le système de nucléase spécifique à un site est :
un système CRISPR/Cas contenant une combinaison d'un ARN guide qui cible les séquences (i) et (ii) et d'un ARN guide qui cible la séquence (iii), ou
un système de nucléase artificiel contenant une combination : d'une protéine de fusion qui contient un domaine de liaison à l'ADN qui cible les séquences (i) et (ii) et un domaine nucléase, et d'une protéine de fusion qui contient un domaine de liaison à l'ADN qui cible la séquence (iii) et un domaine nucléase,
dans lequel le procédé intègre l'acide nucléique donneur par la réparation combinatoire d'une jonction d'extrémités non homologues des séquences clivées (i) et (ii) et une recombinaison homologue du bras d'homologie non clivé avec la séquence génomique, dans lequel le procédé n'est pas un processus visant à modifier l'identité génétique de lignée germinale des êtres humains.

2. Utilisation d'un kit ou d'une composition dans le procédé de la revendication 1, dans lequel le kit ou la composition comprend :
un système de nucléase spécifique à un site et un ADN donneur, dans lequel
l'ADN donneur est un ADN contenant une séquence nucléotidique dans laquelle une séquence de bras d'homologie côté 5', une séquence donneuse, et une séquence de bras d'homologie côté 3' sont agencées dans cet ordre à partir du côté 5',
le système de nucléase spécifique à un site est un système qui cible et clive les séquences (i) à (iii) du groupe A ou du groupe B ci-dessous, et les séquences (i) et (ii) sont ciblées par les mêmes molécules constituant le système de nucléase spécifique à un site, et la séquence (iii) est ciblée par d'autres molécules constituant le système de nucléase spécifique à un site,
Groupe A :
(i) la séquence de bras d'homologie côté 5'
(ii) une séquence dans la région cible d'édition génomique correspondant à la séquence (i)
(iii) une séquence dans la région cible d'édition génomique entre une séquence correspondant à la séquence de bras d'homologie côté 5' et une séquence correspondant à la séquence de bras d'homologie côté 3'
Groupe B :
(i) la séquence de bras d'homologie côté 3'
(ii) une séquence dans la région cible d'édition génomique correspondant à la séquence (i)
(iii) une séquence dans la région cible d'édition génomique entre une séquence correspondant à la séquence de bras d'homologie côté 5' et une séquence correspondant à la séquence de bras d'homologie côté 3'
dans lequel le système de nucléase spécifique à un site est :
un système CRISPR/Cas contenant une combinaison d'un ARN guide qui cible les séquences (i) et (ii) et d'un ARN guide qui cible la séquence (iii), ou
un système de nucléase artificiel contenant une combination : d'une protéine de fusion qui contient un domaine de liaison à l'ADN qui cible les séquences (i) et (ii) et un domaine nucléase, et d'une protéine de fusion qui contient un domaine de liaison à l'ADN qui cible la séquence (iii) et un domaine nucléase.
